# EUROPEAN PATENT APPLICATION

(11) **EP 1 083 235 A2**
(43) Date of publication of application: **14.03.2001**
(21) Application number: 00118757.4
(22) Date of filing: 30.08.2000
(51) Int. Cl.: C12Q 1/26, C12Q 1/28, C12Q 1/32, C12Q 1/52

(54) **Reduced coenzyme solution**

(30) Priority: 31.08.1999 JP 24464399
(71) Applicant: ORIENTAL YEAST CO., LTD., Tokyo (JP)
(72) Inventor: Minato, Hideki, Niiza-shi, Saitama-ken (JP); Matsukawa, Hirokazu, Tokyo (JP); Kohda, Seiichi, Kawanishi-shi, Hyogo-ken (JP); Oka, Osamu, Kawagoe-shi, Saitama-ken (JP)
(74) Representative: Leson, Thomas Johannes Alois, Dipl.-Ing.

(57) **Abstract**

There is provided a reduced coenzyme solution which is stable upon storage for a long period; the said solution contains an active oxygen remover.

## Description

### Detailed Description of the Invention:

The present invention relates to stabilization of a solution of a reduced coenzyme upon storage. The present invention is characterized in containing superoxide dismutase and/or catalase which are/is active oxygen remover(s) and in being in a state of a solution.

The present invention further relates to a method for stabilization of a reduced coenzyme upon storage containing an active oxygen remover.

The present invention furthermore relates to a method for the measurement of the components contained in the sample using a reduced coenzyme solution containing an active oxygen remover and also to a reagent therefor.

### Prior Art:

In the past, many liquid reagents for the measurement in clinical test used unstable enzymes and substrates and were supplied as freeze-dried products taking their stabilization into consideration. In recent years, stable liquid reagents which can be used in a state of a solution *per se* have been developed and many of them have been available in the market as easily usable reagents avoiding the operation of dissolving the freeze-dried powdery measuring reagents for each use at the busy places where clinical tests are carried out. However, as compared with the freeze-dried reagents, liquid reagents are difficult to ensure the stability upon storage and there has been a demand for better stabilizing method.

In the fields of clinical chemistry, biochemistry, food chemistry, pharmacy and others, many kinds of enzymatic reactions are used with an object of analysis. Especially in clinical tests, a lot of metabolites and enzymes in blood, serum, plasma, body fluid and urine samples are enzymatically measured and studied. For example, substances which are the components included in living substances such as aspartic acid amino transferase (AST), alanine aminotransferase (ALT), lactate dehydrogenase (LDH), α-hydroxybutyrate dehydrogenase (HBD), non-esterified fatty acid (NEFA), urine nitrogen (UN) and creatinine (CRE) are the routinely measured items as important indexes for diagnosis of various diseases. Reagents for the measurement of the above-mentioned components contain reduced nicotinamide adenine dinucleotide (NADH) or reduced nicotinamide adenine dinucleotide phosphate (NADPH) as a common coenzyme and, in preparing the liquid reagent, it is necessary that the said NADH or NADPH is kept in a state of a solution for a long period in a stable manner.

NADH which is one of the coenzymes participating in an oxygen-reduction enzyme carries out the reaction as expressed by the following formula (1). 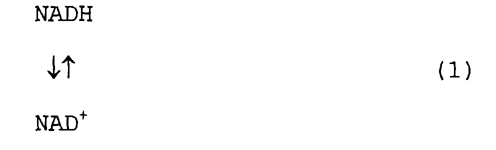 Among the coenzymes which are most abundantly present in the body, NADPH acts as a reducing agent for synthetic reaction *in vivo* while NADH has a significance of acquisition of energy by oxidation of various substrates. Usually, the above oxidation-reduction reaction is reversible. Since NADH has a maximum absorption at about A340 (A338 nm, molar extinction coefficient at pH 9.5 (ε) = 6220), changes in absorbance of the spectrophotometer at A340 are applied to the measurement of various enzymatic activities. Thus, a decrease in the value at A 340 nm due to the change from NADH to NAD⁺ is measured. In external diagnostic agents, various measurements utilizing such a principle are carried out. When a measurement is carried out using the principle of the above formula (1), stability of the reduced coenzyme affects the measurable range and the expiry date of the reagent.

Usually, when a reduced coenzyme such as NAD(P)H (reduced nicotinamide adenine dinucleotide (phosphate)) is stored in a form of a solution, its stability has been improved by storing in a solution of an alkaline condition. Further, with regard to a method for the stabilization of NAD(P)H, a report where a specific base (an amine base such as imidazole or a strong base such as sodium hydroxide) is made coexisted (Japanese Laid-Open Patent Publication Sho-55/42599-A), a report where a chelating agent such as EDTA and an azide compound such as sodium azide are added (Japanese Laid-Open Patent Publication Sho-59/82398-A), a report where boric acid is added (Japanese Laid-Open Patent Publication Sho-62/198697-A), a report where an alkaline metal or ammonium bicarbonate buffer is made coexisted (Japanese Laid-Open Patent Publication Hei-04/229192-A), etc. are available.

However, although NAD(P)H is stable in a solution of an alkaline condition, it is oxidized, though gradually, by various factors such as a coexisting contaminants, concentration of the buffer and changes in pH with a lapse of time. For storage, it is possible to prepare that, taking the decreasing velocity of NAD(P)H into consideration, the adding amount of NAD(P)H is previously increased so that the NAD(P)H in an amount necessary for the desired reaction upon actual measurement can be ensured but such a method is not preferred in terms of cost.

In addition, some enzymes used for the measurement result in the production of inhibitors during the storage of the solution and, further, they are inconvenient for the reagent for the measurement of changes in absorbance of NAD(P)H at A 340 nm. Moreover, in addition to NAD(P)H, the reagent for measuring the components in the sample contains enzymes and substrates necessary for carrying out the desired reaction and other compositions as well. It is of course necessary to keep the optimum conditions (such as a pH range) during the reaction and to take the stability of other compositions into consideration. Thus, it is not satisfactory to just consider in a stabilizing method which is optimum to NAD(P)H only.

In the conventional stabilizing methods as mentioned above, stability of a reduced coenzyme for a long period is insufficient especially in a state of a solution and there is a limit for the stability achieved by such conditions. Thus, no product which is suitable in terms of the desired stability, particularly a solution which is stable upon storage for a long period, has been obtained yet.

### Mean for Solving the Problems:

The present invention provides a reagent solution where reduced coenzyme can be stored in a state of a solution for a long period by containing an active oxygen remover and also a method therefor.

The prevent invention further provides a reagent and a method whereby the components contained in the sample are measured using the above-mentioned reagent.

### Brief Explanation of Drawings:

Fig. 1 shows a stability upon storage of NADH containing superoxide dismutase and catalase.
Fig. 2 shows the result of the measurement of ALT containing superoxide dismutase and catalase.

### Means for Solving the Matters:

The present inventors have carried out an intensive study for solving the above-mentioned conventional problems and found that active oxygen remover maintains the reduced coenzyme in a solution with a high preservability for a long period. It has been also found that the reagent of the present invention can be applied to the measuring system for the substance utilizing an oxidation-reduction reaction of coenzyme whereby the present invention has been accomplished. Thus, the present invention is characterized in using an active oxygen remover in a solution where reduced coenzyme is stored in a stable manner for a long period. In an embodiment, the active oxygen remover in the reduced coenzyme solution of the present invention is superoxide dismutase and/or catalase. In an embodiment, the reduced coenzyme in the reduced coenzyme solution of the present invention is NADH and/or NADPH. The present invention also provides a method where the reduced coenzyme in a form of a solution is made stable upon storage for a long period by containing an active oxygen remover.

The present invention furthermore relates to a reagent and a method whereby the components contained in the living body substance are measured using the above-mentioned reagent of the present invention.

### Reduced Coenzyme Solution

Although the reduced coenzyme which is an object of the present invention is not limited, it covers NADH, NADPH, APADH (reduced 3-acetylpyridine-adenine dinucleotide), APADPH (reduced 3-acetylpyridine-adenine dinucleotide phosphate), etc. In order to keep the stability of the present reduced coenzyme during storage and/or reaction, it is at least necessary to be alkaline, preferably pH 8-11 or, more preferably, pH 9-10 and also to contain an active oxygen remover. In order to keep the alkalinity of the present reagent, it is preferred to contain sodium hydrogen carbonate, sodium carbonate, tris(hydroxymethyl)aminomethane and other buffer. With regard to the concentration of the buffer, it may be 5mM∼50mM in the case of tris(hydroxymethyl)aminomethane for example.

Usually, there is no limitation for the active oxygen remover so far as it is a substance which removes oxygen species of active oxygen such as superoxide (O₂⁻), hydrogen peroxide (H₂O₂), a hydroxyl radical (.OH) and a singlet oxygen (1 O₂). Its examples are superoxide dismutase, catalase, cytochrome C, lactoperoxidase, D-mannitol, tryptophan, α-tocopherol and ascorbic acid. Each of them may be used solely or two or more of them may be used jointly. The adding concentration may be appropriately selected and used.

A superoxide dismutase which is one of the active oxygen removers of the present invention is called a superoxide disproportionating enzyme as well and is an enzyme catalyzing the disproportionating reaction of a superoxide anion radical (O₂ ^{· -}) expressed by the following formula (2). 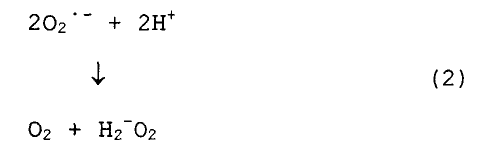 Its example is an enzyme number 1.15.1.1. It is widely present in aerobes and is also found in some kinds of facultative anaerobes and strict anaerobes. Three kinds of enzymes, i.e. an enzyme containing copper and zinc, an enzyme containing manganese and an enzyme containing iron in active center, are known. The superoxide dismutase of the present invention can be extracted from the above-mentioned cells by known methods and purified although unlimited thereto. It may be also a recombinant enzyme obtained by means of gene technology. It has an effect of removing the active oxygen at 0.05 U/ml or more and 0.1-30 U/ml is particularly preferred.

Catalase which is one of the active oxygen removers of the present invention is an enzyme which catalyzes the decomposition reaction of hydrogen peroxide represented by the following formula (3) and its example is 1.11.1.6. 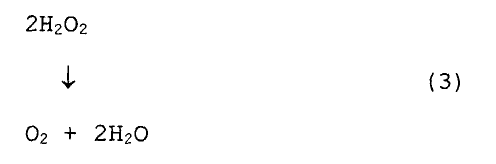 It is widely available in aerobic cells of animals, plants and microbes. The catalase of the present invention can be extracted from the above-mentioned cells by known methods and purified although unlimited thereto. It may be also a recombinant enzyme obtained by means of gene technology. It has an effect of removing the active oxygen at 0.05 U/ml or more and 0.1-10 U/ml is particularly preferred. When this catalase is used, it is more preferred to use it together with the above-mentioned superoxide dismutase.

### Stabilizing method for reduced coenzyme

In the present invention, the reduced coenzyme solution contains the active oxygen remover. To be more specific, it relates to a method for removal of active oxygen by adding an active oxygen remover and reacting with the active oxygen existing in a reduced coenzyme solution. Thus, for example, there is provided a method wherein alanine, sodium azide and lactate dehydrogenase are dissolved in a tris buffer (pH 9.2) containing NAD(P)H and then superoxide dismutase and/or catalase are/is applied thereto in any order or at the same time whereby the active oxygen is removed.

### Measuring Method using Reduced Coenzyme Solution

There is no particular limitation for the composition of the liquid reagent for measuring the components contained in the sample using the reduced coenzyme solution of the present invention and various components such as dehydrogenase may be selected depending upon the measuring system. Such a combination is also able to be generally used in the measuring liquid reagent using an enzymes such as glucose-6-phosphate dehydrogenase, isocitrate dehydrogenase, glutamate dehydrogenase, alanine amino transferase (ALT) and aspartic acid amino transferase (AST). Specific adding amount, substrate and other compositions may be appropriately selected depending upon each measuring system used. Further, the liquid reagent of the present invention will do provided that a reduced coenzyme solution contains an active oxygen remover and other substances in the liquid reagent and there is no limitation for the adding stage.

Although there is no particular limitation for the sample in the present invention, its examples are any substance which is an object for a clinical test existing in organic samples such as blood, serum, plasma, urine, spinal fluid, cell or tissue extract, saliva and sweat, e.g. various enzymes including aspartic acid amino transferase, alanine amino transferase, lactate dehydrogenase, α-oxybutyrate dehydrogenase, non-esterified fatty acid, urea nitrogen and creatinine.

With regard to a measurement using, for example, ALT as an embodiment of the present invention, a commonly used method may be used and there is no limitation. According to a method recommended by the JSCC for example, 0.3 ml of serum is added to and mixed with 2.4 ml of a reagent 1 consisting of superoxide dismutase, NADH, lactate dehydrogenase, L-alanine, catalase and tris hydrochloride buffer (pH 9.2; 30°C) and the mixture is kept at 30°C for 8 minutes. To the said mixed solution is added 0.3 ml of a reagent 2 of tris hydrochloride buffer (pH 3.5; 30°C) containing 2-oxoglutaric acid and L-alanine followed by mixing and the mixture is made to react at 30°C for 1 minutes. Absorbance of A 340 nm is measured at 30°C for 2 minutes.

With regard to a measurement using, for example, AST as an embodiment of the present invention, a commonly used method may be used and there is no limitation. According to a method recommended by the JSCC for example, 0.3 ml of serum is added to and mixed with 2.4 ml of a reagent 1 consisting of superoxide dismutase, NADH, lactate dehydrogenase, malate dehydrogenase, L-aspartic acid, catalase and tris hydrochloride buffer (pH 9.2; 30°C) and the mixture is kept at 30°C for 8 minutes. To the said mixed solution is added 0.3 ml of a reagent 2 of Tris hydrochloride buffer (pH 4.0; 30°C) containing 2-oxoglutaric acid and L-aspartic acid followed by mixing and the mixture is made to react at 30°C for 1 minutes. Absorbance of A 340 nm is measured at 30°C for 2 minutes.

The present invention will now be further illustrated by way of the following examples although they are not intended to limit the technical coverage of the present invention thereto. The persons skilled in the art may easily modify and change the present invention based upon the description of the present specification and such modifications and changes are covered within a technical coverage of the present invention.

### Examples:

### Example 1. Stabilization of Reduced Coenzyme NADH

A preparation solution having the following composition was prepared using NADH which is one of the reduced coenzymes, each 10 ml of the solution were placed in tightly sealed glass container and the absorbance at 340 nm was measured immediately after preparation and also after one, two and three month(s) under the following condition whereby stability of the reduced coenzyme upon stability was tested. In this test, data after each storing period were calculated on the basis of the data immediately after preparation which was set as 100% and shown as residual rates of the reduced coenzyme. With regard to a control, the test was carried out without adding the active oxygen remover. When the active oxygen remover was added, a case where superoxide dismutase and catalase were jointly used and a case where only superoxide dismutase was added were carried out.

### (Composition)

| | |
|---|---|
| Tris hydrochloride buffer | 20 mM |
| Alanine | 500 mM |
| Sodium azide | 0.1 % |
| Lactate dehydrogenase | 0.25 U/ml |
| NADH | 0.2 mM |

| Active oxygen remover | |
|---|---|
| Superoxide dismutase | 30 U/ml |
| Catalase | 1.0 U/ml |

### (Condition)

| | |
|---|---|
| Storing temperature: | 11 °C |
| Measuring wave length: | 340 nm |
| pH of stored solution: | 9.2 (30°C) |

With regard to activity unit, the amount for producing 1 µmol of NAD⁺ per minute was defined as one unit.

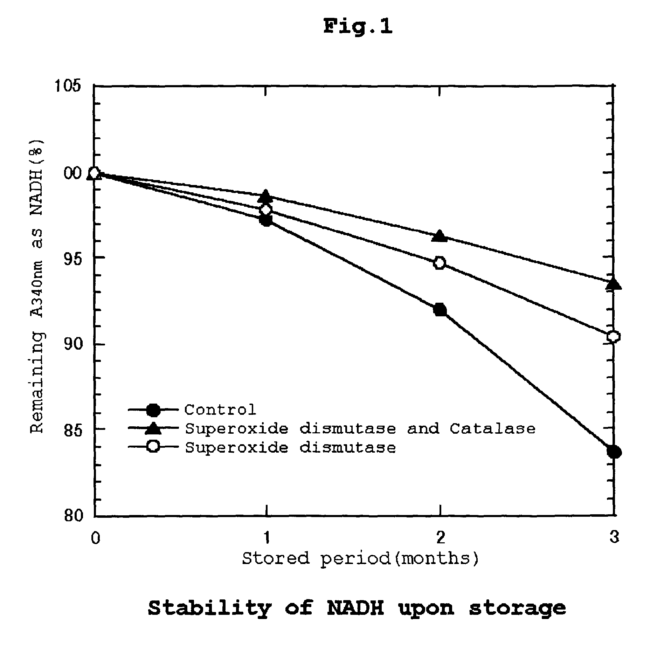

The result is shown in Fig. 1. It was found as a result that, in the case of the reduced coenzyme solution of the present invention using superoxide dismutase and catalase as active oxygen removers, the residual rate of NADH even after stored at 11°C for three months from the preparation was nineties % and, as compared with the control, it was found to be stable for storage in a form of a solution.

### Example 2. Measurement of ALT using Reduced Coenzyme Solution

Measurement of ALT was carried out using a reduced coenzyme solution. After ALT of 1,200 U/liter was diluted in 11 stages, each 15 µl thereof were added to a solution where 240 µl of the reagent 1 and 120 µl of the reagent 2 were previously mixed and the reaction was carried out at 37°C to measure the ALT activity. With regard to an active oxygen remover in the composition, a case where superoxide dismutase and catalase were jointly used and a case where only superoxide dismutase was used were carried out.

### (Composition)

### Sample:

Pure ALT derived from human being (manufactured by Aalto)

### Reagent 1:

| | |
|---|---|
| Tris hydrochloride buffer (pH 7.5; 30°C) | 111 mM |
| NADH | 0.2 mM |
| Lactate dehydrogenase | 2500 U/liter |
| L-Alanine | 625 mM |

| Active oxygen remover | |
|---|---|
| Superoxide dismutase | 30 U/ml |
| Catalase | 1.0 U/ml |

### Reagent 2:

| | |
|---|---|
| Tris hydrochloride buffer (pH 7.5; 30°C) | 111 mM |
| 2-Oxoglutaric acid | 150 mM |

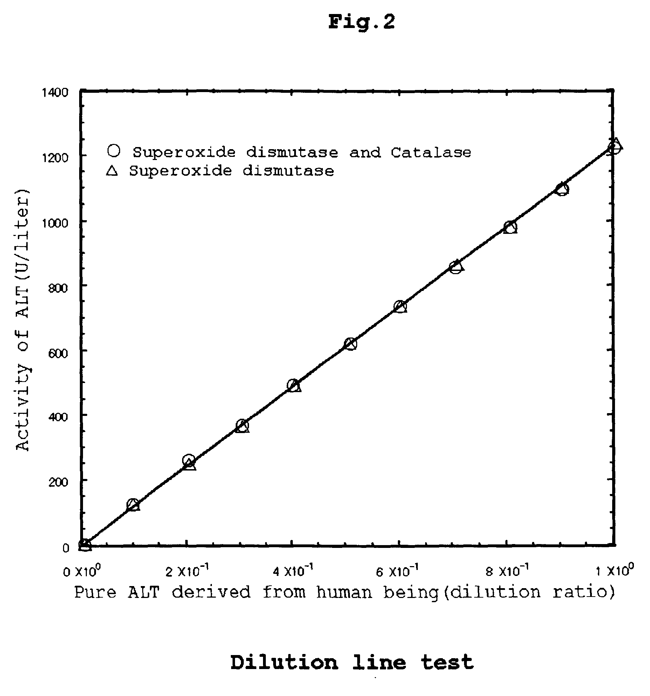

The result is shown in Fig. 2. It was found as a result that the reduced coenzyme solution of the present invention using superoxide dismutase and catalase as active oxygen removers did not affect at all the measurement of ALT which was used in the actual measuring system.

### Advantage of the Invention

As mentioned above, the reduced coenzyme solution of the present invention containing an active oxygen remover keeps the high stability in the reduced coenzyme solution for a long period and is able to provide a reagent which does not affect the measurement at all.

There is provided a reduced coenzyme solution which is stable upon storage for a long period; the said solution contains an active oxygen remover.

## Claims

1. A reduced coenzyme solution containing an active oxygen remover.

2. The reduced coenzyme solution according to claim 1, wherein the active oxygen remover is a superoxide dismutase and/or catalase.

3. The reduced coenzyme solution according to claim 1, wherein the reduction type coenzyme is NADH and/or NADPH.

4. A method for the stabilization of a reduced coenzyme solution in which the reduced coenzyme is stabilized by the active oxygen remover mentioned in any of claims 1 and 2.

5. In a measuring liquid reagent using at least a reduced coenzyme, a liquid reagent for measurement containing an active oxygen remover mentioned in any of claims 1 and 2.

6. The liquid measuring reagent according to claim 5, wherein the said reagent is a liquid reagent for the measurement of alanine amino transferase.

7. The liquid reagent for the measurement of alanine amino transferase according to claim 6, wherein the said reagent contains superoxide dismutase, NADH, lactate dehydrogenase, L-alanine and catalase.

8. The liquid measuring reagent according to claim 5, wherein the said reagent is a liquid reagent for the measurement of an aspartic acid amino transferase.

9. The liquid reagent for the measurement of an aspartic acid amino transferase according to claim 8, wherein the said reagent contains superoxide dismutase, NADH, lactate dehydrogenase, malate dehydrogenase, L-aspartic acid, catalase Tris hydrochloride buffer.
